# EUROPEAN PATENT APPLICATION

(11) **EP 4 621 799 A1**
(43) Date of publication of application: **24.09.2025**
(21) Application number: 24164574.6
(22) Date of filing: 19.03.2024
(51) Int. Cl.: G16H 40/63

(54) **DEVICE, SYSTEM AND METHOD FOR PERSONALIZED NOISE PREPARATION OF A PATIENT SCHEDULED TO UNDERGO A MEDICAL PROCEDURE**

(71) Applicant: Koninklijke Philips N.V., 5656 AG Eindhoven (NL)
(72) Inventor: SCHADEWALDT, Nicole, Eindhoven (NL); NAUTS, Sanne, Eindhoven (NL)
(74) Representative: Philips Intellectual Property & Standards

(57) **Abstract**

The present invention relates to a device for personalized noise preparation of a patient scheduled to undergo a medical procedure, the device comprising an input configured to obtain procedural information comprising information about the scheduled
medical procedure; a processor configured to obtain and/or generate sound sequence information comprising information about sound sequences associated with sequences of the scheduled medical procedure based on the obtained procedural information, select and order the sound sequence information, particularly in accordance with the course of the medical procedure, based on the procedural information; and generate control signals for controlling a rendering unit to render the sound sequences in accordance with the selected and ordered sound sequence information; and an output configured to output the control signals to the rendering unit.

## Description

### FIELD OF THE INVENTION

The present invention relates to device, system and method for personalized noise preparation, particularly mental noise preparation, of a patient scheduled to undergo a medical procedure. The medical procedure may generally be any medical procedure involving noise, particularly a computed tomography (CT) scan or magnetic resonance imaging (MRI).

### BACKGROUND OF THE INVENTION

Medical examinations are anxiety-provoking for most patients due to the frightening clinical situation and the prospect of a negative outcome involved. A further factor contributing to anxiety is loud, unpleasant and unpredictable noise in medical procedures, which very specifically applies to MRI, for example. MRI sounds can be very loud (up to 117 dB; Counter et al., 1997) and have been described by patients as "being inside a (-) fax machine" (Hewis, 2015).

Accordingly, such noise induces a significant level of stress for the patient, both before and during an exam. Specifically, the noise leads to uneasiness during the exam with potential needs for scan aborts, retakes, or involuntary movement resulting in image degradation, and a negative feeling of the patient after the scan. The latter will make it even more difficult to scan the same patient a second time, or to scan new patients who have heard negative accounts of the disturbing sound experience of such exams.

Currently, most patients get a description for the noise-experience, e.g. "a knocking sound", as put on one webpage. This does not at all convey, however, what the noise sounds like, and describes only a fraction of the sequence. The patients might themselves google for MRI or other noises and be presented a sequence, however, that gives them no idea of what their individual sequence might sound like.

### SUMMARY OF THE INVENTION

It is an object of the present invention to further improve preparation of a patient scheduled to undergo a medical procedure involving noise, particularly an MR scan. Specifically, the stress level created by the noise of these procedures shall be reduced and negative associations with the noise shall be removed.

In a first aspect of the present invention a device for personalized noise preparation of a patient scheduled to undergo a medical procedure is presented, the device comprising:
an input configured to
obtain procedural information comprising information about the scheduled medical procedure;
a processor configured to
   - obtain and/or generate sound sequence information comprising information about sound sequences associated with sequences of the scheduled medical procedure based on the obtained procedural information;
      - select and order the sound sequence information, particularly in accordance with the course of the medical procedure, based on the procedural information; and
      - generate control signals for controlling a rendering unit to render the sound sequences in accordance with the selected and ordered sound sequence information; and
an output configured to output the control signals to the rendering unit.

In a further aspect of the present invention a system for personalized noise preparation of a patient scheduled to undergo a medical procedure is presented, the system comprising:
a device for personalized noise preparation of a patient scheduled to undergo a medical procedure as disclosed herein; and
a rendering unit configured to render sound sequences according to the control signals obtained from the device.

In yet further aspects of the present invention, there are provided a corresponding method, a computer program which comprises program code means for causing a computer to perform the steps of the method disclosed herein when said computer program is carried out on a computer as well as a non-transitory computer-readable recording medium that stores therein a computer program product, which, when executed by a processor, causes the method disclosed herein to be performed.

Preferred embodiments of the invention are defined in the dependent claims. It shall be understood that the claimed method, system, computer program and medium have similar and/or identical preferred embodiments as the claimed device, in particular as defined in the dependent claims and as disclosed herein.

The present invention is based on the idea to familiarize the patient with the noise of the medical procedure he/she is schedule to undergo and to give them a level of control over it. The patient shall be provided with a preview of his/her personal procedure journey by providing sound sequences associated with the sound sequences of the procedure. Accordingly, the patient can enter the medical procedure knowing what sounds to expect. In other words, the noise is made predictable for the patient. This will significantly reduce the patient's stress level. Negative associations with the noise during the scheduled medical procedure are removed. Thus, aborts and re-scans can be avoided.

The procedural information used to obtain/generate the sound sequence information or, in other words, the sound sequences the patient will hear during the medical procedure, includes details of the medical procedure, so that the respective sound sequences (sound sequence information) can be obtained. For example, the procedural information may indicate that the medical procedure involves an MRI scan (and particularly the MRI sequences to be run) or a CT scan (and particularly the CT sequences to be run). Accordingly, sounds corresponding to the MRI scan or a CT scan, respectively, can be obtained and/or created. In other words, first, the medical procedure the patient is scheduled for is analyzed. For example, the duration of MR exam card components is determined and based on said analysis the sounds and volume associated with each component is determined. These sounds can then be presented to the patient for familiarizing him/her with the noises he/she can expect to hear during the procedure.

Compared to a generic one-size-fits-all app, an app (or, more generally, a device and method as presented herein) that is tailored and personalized will do a better job of preparing patients for their medical procedure. It can prepare patients for exactly those noises that are relevant to them. It can provide a personalized experience that prepares patients for exactly those noises that they need to know/master to the extent that they need to master them.

According to an embodiment, the processor is configured to select and order the sound sequence information, based on the procedural information, to generate the control signals for controlling the rendering unit to render the sound sequences to match the acoustic pattern, pitch, pace, length and/or order of sound sequences generated by a medical device used in the course of the scheduled medical procedure.

Hence, the patient may be provided with a preview of his/her scheduled medical procedure with the exact sequences and duration of the planned procedure, for example. In particular, the patient may not only be provided with random sequences of sounds during an MRI, but with the exact sound sequences of his/her scheduled procedure. The preparation of the patient to the medical procedure is thus highly personalized.

According to an embodiment, the processor is configured to obtain calibration information, comprising information for calibrating the processor to adapt the sound sequence information in accordance with specific sound sequence features related to specifics of the medical device used in the course of the medical procedure, the specifics comprising any one of type, age, condition, use frequency, environment of the medical device, and/or in accordance with sample sounds from the medical device, and to adapt the sound sequence information based on the calibration information.

In particular, the calibration information may comprise said specifics.

This way, the patient can be prepared to the upcoming medical procedure in an even more personalized/exact manner. In fact, MRI sounds, for instance, differ between machines, for example, with respect to pitch and pace. This may be caused by differences in type or age of the machine. However, other specifics are likewise conceivable. The environment of an MRI machine, particularly the room where such a machine is located, plays a role with respect to possible resonances. Hence, calibrating the device to generate sound sequences matching an MRI system's exact noises will give the patient a better impression of what sounds to expect during his/her medical procedure.

The calibration information preferably comprises information about the specifics of the medical device(s) used in the (course of the) medical procedure, the specifics comprising any one of type, age, condition, use frequency, environment of the medical device(s) used.

Preferably, the processor is configured to obtain the calibration information from a user interface configured to obtain user input and/or from a calibration information database. However, the calibration information may likewise be part of the procedural information. The calibration may be done in the room of the medical procedure, i.e. an MRI room, by running (specified) sample sound sequences and recording the sound.

In another embodiment, the processor is configured to obtain sound manipulation information, comprising information about a request of the patient for manipulation of the sound sequences, wherein the sound manipulation information comprises information on any one of changing volume, acoustic pattern, pitch, pace, length, selection and/or order of one or more of the sound sequences, and to adapt the sound sequence information in accordance with the sound manipulation information. Said sound manipulation would preferably only be used in the patient preparation phase, not in the final medical examination phase.

Accordingly, the patient may familiarize himself/herself with the sound of the medical procedure in a controllable way, for example, by controlling the volume or the order of the sound sequences. Sound sequences perceived as unpleasant may thus be listened to repeatedly to familiarize with said sound and to reduce stress and/or anxiety associated with said sound.

Preferably, the processor is configured to obtain the sound manipulation information from a user interface configured to obtain user input.

In another embodiment of the device, the processor is further configured to obtain and/or generate one or more entertainment program components, comprising one or more of an audio entertainment component, a video entertainment component and/or an interactive entertainment component, based on the procedural information, and to generate entertainment control signals for controlling the rendering unit to render the one or more entertainment program components based on the entertainment program components;
wherein the output is configured to output the entertainment control signals to the rendering unit.

For example, the processor may obtain and/or generate multiple video-audio-journeys to accompany the sound sequences. Particularly, sound sequences of the medical procedure being very loud may be accompanied by a soothing video entertainment component, for example a video of the sea or the like, and/or by a soothing audio component, e.g. soothing music. Thus, using the device presented there can be created a so-called ambient experience (AE) for the patient, i.e. a soothing and comforting environment, in which stress, anxiety and discomfort are reduced.

In general, the processor may obtain and/or generate the one or more entertainment program components based on patient information instead of procedural information.

For generating the entertainment program components various types of technology may be used, including artificial intelligence. For example, there may be provided an "exam card specific journey generator". In other words, the processor may be configured to generate entertainment program components in accordance with information stored on an exam card of the patient. Preferably, the processor is configured to generate entertainment program components, particularly, sequences of video and/or audio themes that fit the scheduled medical procedure, particularly in length of the components/sequences and type. For example, the processor may generate (or obtain) abstract video and audio scenarios matching MR sounds, e.g. falling balls, or MR specific video and audio scenarios, e.g. jack-hammer, angle-grinder, stampede of horses/wildbeasts, rock-music scene, instrumental scenes. Similarly, the processor may generate (or obtain) a game that is accompanied by the sound sequences of the medical procedure, for example, in which the patient can use an avatar that can interact with the sounds (e.g., interacting with characters that move in the rhythm of the sound; creating music that accompanies the sound, etc.).

With the term "entertainment program components" components and/or sequences of an entertainment program, including a video entertainment program, an audio entertainment program and/or an interactive entertainment program component are meant. Accordingly, an entertainment program component may comprise a scene / sequence of a film or other video entertainment and/or a sequence of a piece of music or other sound and/or a sequence of an interactive game or conversation (e.g. with an AI-generated answering machine) or the like. In this context, the term "entertainment" shall be understood broadly. Accordingly, a video entertainment component may be any visualization of the sound sequences, e.g. a visual representation of sound waves corresponding to the sound sequences or a representation of physical objects to represent sound frequencies.

The processor may be configured to select and/or order the video, audio and/or sound components in accordance with the procedural information, particularly in accordance with the course of the scheduled medical procedure.

Preferably, the processor is configured to obtain the one or more entertainment program components from a user interface configured to obtain user input and/or from an entertainment program components database, the entertainment program components database comprising an ambient experience library or another library comprising entertainment program components, i.e. entertainment content, particularly audio/visual (and possibly interactive) content.

In an embodiment, the processor is configured to obtain entertainment manipulation information comprising information about a request of the patient for manipulation of the entertainment program components, wherein the entertainment manipulation information comprises information on any one of changing type, theme, volume, acoustic pattern, pitch, pace, length, selection and/or order of the one or more audio entertainment components, changing any one of type, theme, display size, resolution, brightness, contrast, coloring, length, selection and/or order of the video entertainment components and/or changing any one of type, theme, level of interactivity, length, selection and/or order of the interactive entertainment components, and to adapt the one or more entertainment program components in accordance with the entertainment manipulation information.

Hence, the patient is given control over the ambient experience of his/her scheduled medical procedure. The patient may thus personalize the course of their medical procedure, such as their MR journey, as desired. They can listen to the sounds of the medical devices used during their procedure, e.g. MR sounds, in advance at a chosen volume, increasing up to 100%. They may be suggested matching video and audio content based on a themes library. The patient may choose (components) alternatives from a (large) library of video, audio and/or interactive themes, edit the video and/or audio content and select different videos from a library. A preview may be available, for instance, where the video-content is played full screen with the audio-content and (MR) sequence noises superimposed.

Given said possibilities, the patient is enabled to exert control over the medical procedure (e.g. MR) experience, e.g. he/she may choose themes that are soothing to him/her, or indicate sequence changes or the end of the exam to himself/ herself with certain themes.

Hence, there is provided a device with which the patient becomes familiarized
with the noises to be expected during his medical procedure and can control the experience by designing his/her personalized accompanying video/audio/interactive experience (i.e. ambient experience). There is created a personalized ambient experience to fit this specific patient's medical procedure (and, preferably, also preferences).

Apart from that, the patients may familiarize themselves with the sounds of the schedule medical procedure when "playing" with the options to configure their journey. They may choose to change the AE theme content with each component/sequence of their procedure to guide themselves through the procedure, or they may choose to select one theme only to stay with what relaxes them most. Also, patients might focus more on the video content during the procedure because they themselves selected it.

The processor may be configured to obtain the entertainment manipulation information from a user interface configured to obtain user input.

In yet another embodiment of the device, the processor is configured to further obtain patient information comprising information regarding the patient, wherein the processor is configured to additionally use the patient information for obtaining, generating and/or adapting the one or more entertainment program components.

The patient information may be information (about the patient) regarding one or more of (the patient's) age, size, weight, gender, health state, medication, medical history, preferences, sensitivity, personality, problems during a prior medical procedure, frequent emotional states, hearing ability, and/or eyesight. The patient information may be obtained from one or more of a user interface configured to obtain user input, an exam card, a medical record, and a patient information database.

While younger patient's generally tend to prefer audio components comprising pop music, elderly people generally tend to prefer classical music. Similarly, woman generally tend to prefer video material with romantic content while men tend to prefer action films. Therefore, the processor may be configured to obtain and/or generate corresponding entertainment program components. For example, the processor may be configured to obtain entertainment program components with a particularly large selection of action films for male patients.

Any one of type, theme, volume, acoustic pattern, pitch, pace, length, selection and/or order of the one or more audio entertainment components, any one of type, theme, display size, resolution, brightness, contrast, coloring, length, selection and/or order of the video entertainment components and/or any one of type, theme, level of interactivity, length, selection and/or order of the interactive entertainment components may be adapted by the processor based on the patient information. For example, if the patient information indicates that the patient is hard of hearing, the volume of the audio entertainment components may the increased. Similarly, video entertainment components such as video films may be displayed with subtitle.

Accordingly, when the patient uses the device (i.e. app on the device), they may be asked to provide some preferences for their personalized journey, which can then be taken into account by the processor (or more specifically "journey generation engine") in preparing them for the noises to expect.

In an embodiment, the obtained one or more entertainment program components comprise one or more labels and/or the processor is configured to assign one or more labels to the obtained and/or generated one or more entertainment program components, wherein the processor is configured to assign the one or more labels by using a label database or a labeling program relating the one or more labels to the one or more entertainment program components, and wherein the processor is configured to select and/or order the one or more entertainment programs based on their labels and to generate the entertainment control signals based on the selected and/or ordered one or more entertainment program components.

Using labels, entertainment components (generally perceived to be) matching the sound sequences of the scheduled medical procedure can be obtained/selected/ordered in an organized and efficient manner. In other words, ambient experience library content may be tagged, to which MR sequence they match well, such that the processor can obtain matching sounds, and that matching sounds appear at a top of a list presented to the patient for editing. The labels may indicate any one of theme, type, genre, length, rating (e.g. by prior patients), content, age restriction, (level of and) emotional state (generally) associated/evoked with the one or more entertainment program components etc. of the entertainment program components.

For example, the labels may indicate levels of an emotional state associated with the audio entertainment components, for example levels of arousal. Similarly, the labels may indicate levels of suspense of the video entertainment components.

It is known that threatening situations like a thunderstorm (in a film, for example) do have a negative and not soothing effect on patients. Thus, preferably, negative scenarios in the entertainment program components should be avoided. Animated scenes are found to be completely neutral. If construction site scenes are used, they should lead to something aesthetic, a building, a sculpture, a playground, a bridge or similar. A stampede should rather be running through water for fun, jumping and playing, and not being hunted by a predator. In any case, the processor shall be configured to arrange the selection and/or order to entertainment program components to ensure that a positive ending is within the duration of the medical procedure, and thus the situation "resolved". To this end, the processor may use the labels as indicator.

In an embodiment, the input of the device is configured to obtain, as procedural information, information regarding one or more of type of medical procedure, medical device(s) used in the medical procedure; body part of the patient to undergo the medical procedure, duration and/or time of the medical procedure, course of the medical procedure.

In another embodiment, the input is configured to obtain the procedural information from one or more of a user interface configured to obtain user input, an exam card, a medical record, a procedural information database.

In another embodiment, the processor is configured to obtain progress information, the progress information indicating the progress of the medical procedure (in real-time), and to adapt the sound sequence information and/or the entertainment program components in accordance with the progress information.

The output of the system, i.e. the patient selected personalized exam experience scenarios comprising possibly entertainment components (such as audio or video entertainment components) may be adapted during the medical procedure. For example, the control signals may be transferred to a rendering unit of a system in a hospital, which is connected with the medical device, e.g. an MR scanner. Thus, in case the medical procedure is differing from the planned medical procedure "control points" may be exchanged and the patient selected exam experience scenario may be adapted, e.g. loop parts that belong to a specific MR sequence may be included if the sequence needs to be repeated, and a start of the next sequence experience may be triggered earlier, if a sequence starts earlier. Alternatively, the rendering unit, or the hospital's AE system, may be equipped with a microphone to observe the progress of the medical procedure and the processor of the device may "align" the pre-programmed personalized exam experience with the real exam progress, in case there should be deviations.

According to an embodiment of the system, the system comprises, in addition to the disclosed device and a rendering unit, one or more of a
- user interface configured to obtain user input of the sound sequence information, procedural information, calibration information, patient information, sound manipulation information and/or entertainment manipulation information;
- a sound sequence information database comprising sound sequence information comprising information about sound sequences associated with sequences of the scheduled medical procedure
- a procedural information database comprising procedural information comprising information about the scheduled medical procedure;
- a calibration information database comprising calibration information, the calibration information comprising information for calibrating the processor of the device to adapt the sound sequence information in accordance with specific sound sequence features related to specifics of the medical device used in the course of the medical procedure;
- a patient information database comprising patient information comprising information regarding the patient;
- an entertainment program components database comprising a plurality of entertainment program components, in particular comprising one or more of an audio entertainment component, a video entertainment component and/or an interactive entertainment component.

The user interface may e.g. comprise a keyboard, touchscreen, microphone or any other entity that allows a user to enter information. In particular, the user interface may be used by the patient to review and modify the ambient experience (sound sequences, entertainment program components) created by the device, e.g. the patient's exam card specific journey. For instance, the user interface may comprise a module to play the sound sequences (e.g. MRI sounds) at modifiable volume; a module to play/reproduce the entertainment program components, e.g. a video-audio-journey, with or without the sound sequences, e.g. MR sound; and/or a module to save the personalized sound sequences and entertainment program components, i.e. the personalized "ambient experience journey" and transfer it to a hospital AE system, particularly a rendering unit of said system.

In an embodiment of the system, the system is implemented in a handheld device, in particular a smartphone, laptop or tablet, and/or in a computer or a game console;
wherein the rendering unit is implemented as a display configured to display visual information to the patient and/or as a loudspeaker configured to output audible information to the patient. The term `handheld device' shall be understood generally as covering any suitable device that is meant to be held by a user when in use, irrespective of whether the patient is actually holding the device or not.

### BRIEF DESCRIPTION OF THE DRAWINGS

These and other aspects of the invention will be apparent from and elucidated with reference to the embodiment(s) described hereinafter. In the following drawings
Fig. 1 shows a schematic diagram of an implementation of a system according to the present invention;
Fig. 2 shows a schematic diagram of an embodiment of a device according to the present invention;
Fig. 3 shows a schematic diagram of an embodiment of a method according to the present invention; and
Fig. 4 shows a schematic diagram of an implementation of a device according to the present invention in the form of a smartphone.

### DETAILED DESCRIPTION OF EMBODIMENT

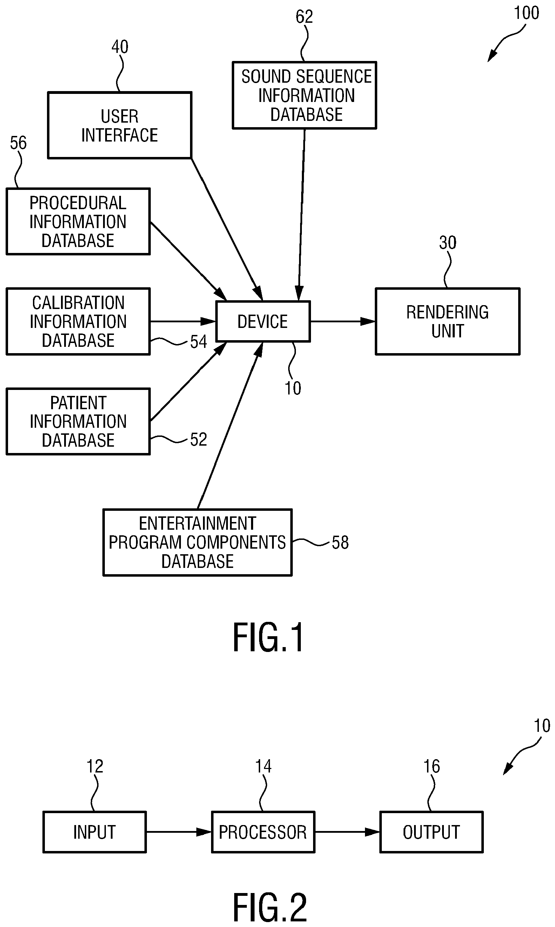

Fig. 1 shows schematic diagram of an embodiment of a system 100 for personalized noise preparation of a patient scheduled to undergo a medical procedure according to the present invention. The system 100 comprises a device 10 personalized noise preparation of a patient scheduled to undergo a medical procedure as will be explained in more detail below and a rendering unit 30 configured to render visual and/or audible information according to control signals received from the device 10.

The system 100 may be implemented in a computer or a game console or a handheld device, in particular a smartphone, laptop or tablet.

The device 10 may generally be implemented by respective units or circuitry, e.g. a processor, processing circuitry, a computer, dedicated hardware, etc., that carries out the functions of the device 10. Alternatively, a common unit or circuitry, e.g. a common processor or computer, may implement the various functions of the device 10, or separate units or elements may be used that together represent the circuitry. In an exemplary implementation, a programmed processor may represent the device 10, which may execute a computer program that may be stored in a memory that is accessed by the processor.

The rendering unit 30 may generally be any means that outputs visual and/or audible information, e.g. in text form, as image or diagram, as sound or spoken words, etc. For instance, the rendering unit 30 may comprise (or be implemented as) a display configured to display visual information to the patient and/or a loudspeaker to output audible information to the patient. Exemplary implementation may use a touchscreen, a computer monitor, the screen of a smartphone or tablet, etc. as rendering unit 30.

The system 100 may further comprise a user interface 40 that is configured to receive user input, e.g. of procedural information, calibration information, patient information, sound manipulation information and/or entertainment manipulation information. The user interface 40 may e.g. comprise a keyboard, touchscreen, microphone or any other entity that allows a user to enter information.

The system 100 may further comprise a sound sequence information database 60 comprising sound sequence information comprising information about sound sequences associated with sequences of the scheduled medical procedure. Preferably, the sound sequence information comprises information about the features of the sounds during the medical procedure necessary to reproduce essentially the exact sound during the procedure.

The system 100 may further comprise a procedural information database 56 containing procedural information comprising information about (medical procedures including) the scheduled medical procedure. The procedural information database 56 may be stored on a server, central storage of a hospital or medical service, in the cloud or at another location that is accessible by the device 10. The procedural information may e.g. be the type of medical procedure (e.g. information about a scan modality (such as MRI or CT), the medical device(s) used in the medical procedure; the body part of the patient to undergo the medical procedure, the duration and/or time of the medical procedure, the course of the medical procedure, e.g. scan sequences, particularly MR sequences.

The system 100 may further comprise a patient information database 52 containing patient information comprising information regarding the patient. The patient information database 52 may be stored on a server, central storage of a hospital or medical service, in the cloud or at another location that is accessible by the device 10.

The system 100 may further comprise an entertainment program components database 58 comprising a plurality of entertainment program components, in particular comprising one or more of an audio entertainment component, a video entertainment component and/or an interactive entertainment component. The video entertainment component may comprise any one of a video component, an image component, a written text component, a movie component. The audio entertainment component may comprise any one of a sound component, a music component, a spoken text component, a movie component. The interactive entertainment component may comprise any one of a game component, a conversation component, a video call component, a phone call component. The entertainment program components database 58 may be stored on a server, central storage of a entertainment program components provider, in the cloud or at another location that is accessible by the device 10. The entertainment program components database 58 may be able to generate further entertainment program components or provide the entertainment program components to another entity for generating further entertainment content components.

The system 100 may further comprise a calibration information database 54 comprising calibration information, the calibration information comprising information for calibrating the processor of the device to adapt the sound sequence information in accordance with specific sound sequence features related to specifics of the medical device used in the course of the medical procedure. The calibration information database 54 may be stored on a server, central storage of a calibration information database provider, in the cloud or at another location that is accessible by the device 10.

Two or more of the databases 52 to 60 may also be combined into common databases and/or stored at the same location, such as the cloud or archive or hospital's network.

The term `handheld device' shall be understood generally as covering any suitable device that is meant to be held by a user when in use, irrespective of whether the patient is actually holding the device or not. The handheld device is preferably a mobile phone as this is a device most people own or have direct access to.

As most people own or have access to a suitable handheld device, they can be supplied with the software to run the noise preparation on their handheld device. If not, then they may be given a mobile device temporarily to use for the training. An advantage is that the patient can do the preparation with the device at a place and time which is convenient for them, for instance at home.

Fig. 2 shows a schematic diagram of a device 10 for personalized noise preparation of a patient scheduled to undergo a medical procedure. The device comprises an input 12 configured to obtain procedural information comprising information about the scheduled medical procedure, a processor 14 configured to obtain and/or generate sound sequence information comprising information about sound sequences associated with sequences of the scheduled medical procedure based on the obtained procedural information, select and order the sound sequence information, particularly in accordance with the course of the medical procedure, based on the procedural information; and generate control signals for controlling a rendering unit 30 to render the sound sequences in accordance with the selected and ordered sound sequence information, and an output 16 configured to output the control signals to the rendering unit 30.

The input 12 may be directly coupled or connected to the user interface 40 and/or to one or more of the databases 52 to 58 in order to obtain (i.e. retrieve or receive) the required information. The information may also be stored in a storage, buffer, network, or bus, etc. The input 12 may thus e.g. be a (wired or wireless) communication interface or data interface, such as a Bluetooth interface, Wi-Fi interface, LAN interface, HDMI interface, direct cable connection, or any other suitable interface allowing information transfer to the device 10.

The processor 14 may be any kind of means configured to process the information and determine control signals there from. It may be implemented in software and/or hardware, e.g. as a programmed processor or computer or app on a user device such as a smartphone, smartwatch, tablet, laptop, PC, workstation, etc.

The output 16 may generally be any interface that provides the determined control signals, e.g. transmits them to another device or provides it for retrieval by another device (e.g. a smartphone, computer, tablet, etc.). It may thus generally be any (wired or wireless) communication or data interface.

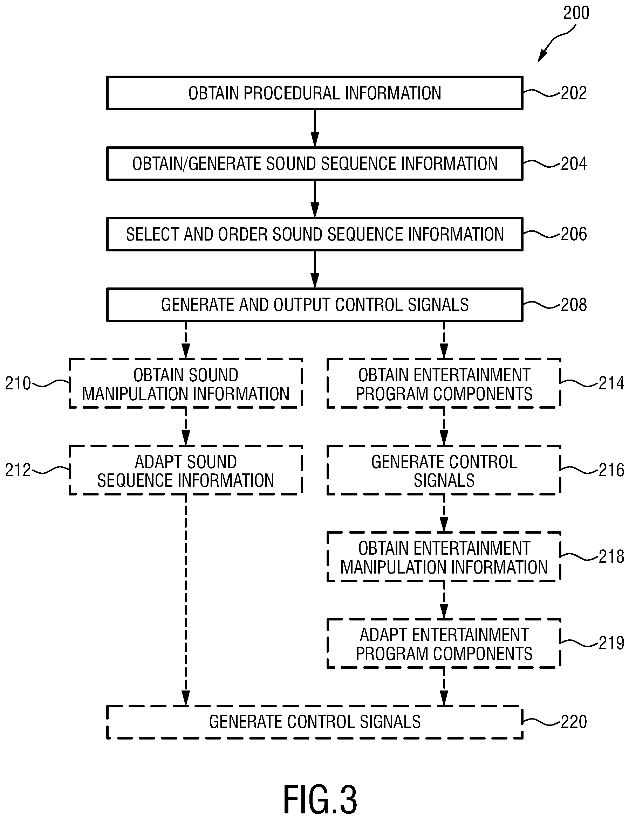

Fig. 3 shows a schematic diagram of an embodiment of a method 200 for personalized noise preparation of a patient scheduled to undergo a medical procedure according to the present invention. The steps of the method 200 may be carried out by the device 10, wherein the main steps of the method 200 are carried out by the processor 14. The method 200 may e.g. be implemented as computer program running on a computer or processor.

In a first step 202 procedural information comprising information about the scheduled medical procedure is obtained (by the input 12 of the device 10). In addition, patient information about the patient and/or calibration information may be obtained.

In a second step 204 sound sequence information comprising information about sound sequences associated with sequences of the scheduled medical procedure is obtained and/or generated in accordance with the procedural information.

In a third step 206 the sound sequence information is selected and ordered, wherein selecting and ordering is preferably performed in accordance with the course of the medical procedure, the course of the medical procedure being indicated by the procedural information.

In a fourth step 208 control signals are generated and output to a rendering unit 30 for controlling the rendering unit 30 to render the sound sequences according to the selected and ordered sound sequence information.

In an optional further step 210 sound manipulation information (from a user interface, for example) may be obtained, the manipulation information comprising information about a request of the patient for manipulation of the sound sequences presented to him/her by the rendering unit 30. The sound manipulation information comprises information on any one of changing volume, acoustic pattern, pitch, pace, length, selection and/or order of one or more of the sound sequences. In accordance with said manipulation information the sound sequence information may then be adapted in a further optional step 212 and corresponding control signals may be generated (and output) in an optional further step 220 so that the rendering unit 30 can render the adapted sound sequences to the patient.

Further optionally, entertainment program components may be obtained (in accordance with the procedural information) in step 214 and corresponding control signals may be generated in step 216 to control the rendering unit to render the entertainment program components the patient. In case the patient requests a change of the entertainment program he/she may use a user interface 40 to provide entertainment manipulation information to the device 10 which then may obtain the entertainment manipulation information in optional step 218, adapt the entertainment program components in accordance with the entertainment manipulation information in optional step 219 and generate corresponding control signals in optional step 220 for controlling the rendering unit 30 to render the adapted entertainment program components.

Generally, the procedural information can be generated through manual input (e.g. from staff), or it can be taken automatically from medical records (e.g., EMR systems), information systems, or other information sources. These information sources can be combined. The procedural information can be automatically pushed to the device, or it can be encoded in a link to an app running on the device 10 (e.g., it can be part of the information provided to patients/their caregivers). For example, patients may receive a (digital) invitation to the medical procedure that includes a QR code containing information about the scheduled procedure.

The sound manipulation information and the entertainment manipulation information may likewise be generated through manual input.

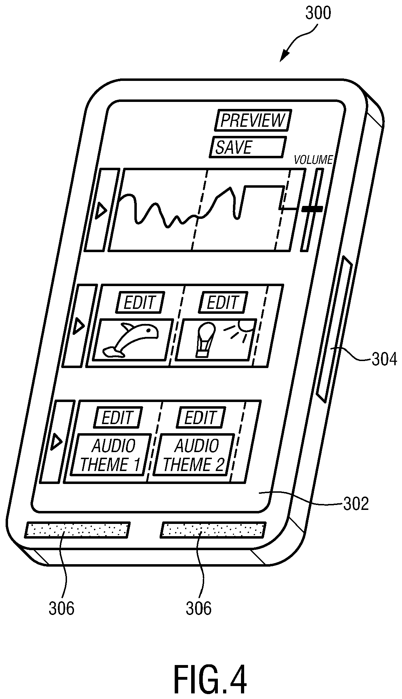

Fig. 4 shows a schematic diagram of an implementation of a device 10 according to the present invention as a handheld device, in particular as a smartphone 300 having a display screen 302, an integrated processor 304, and loudspeakers 306.
The mobile phone 300 may be of any size, shape, model, etc., as long as it is suitable to handle the requirements of being used as a device as disclosed herein.

The display screen 302 may be of any size or type (e.g. LCD, LED and the like). Screens with a large surface area and the capability of displaying strong colors or small and precise details are preferential. Preferably, the display screen 302 is integrated in the smartphone 300, but the visual content may also be duplicated or transmitted to an external display screen connected by wire or wirelessly to the rest of the handheld device, for instance a remote display screen, goggles or glasses, or any virtual reality device (e.g. virtual reality glasses). An integrated screen is the most practical and compact solution, but an external screen would still be part of the handheld device, even though that external screen may not be meant to be handheld or portable itself. Such an external screen may be easier to use in case the patient is not able to watch an integrated screen, e.g. due to the patient's position or other reason that prevents the patient from holding the handheld device and watching the integrated screen at the same time.

The processor 304 shall be capable of receiving, processing and analyzing incoming information and outputting visual and/or audio information, in real time. The processor 304 may also be in contact with a remote processor, for instance through the internet or through a wired or wireless, such as Bluetooth or Wi-Fi, connection with a local external processor, e.g. a workstation that may support the processor in processing power and/or may provide additional information, such as patient data and/or may store data.

It is preferable that the smartphone 300 is capable of providing audio information, such as sound effects, music, coaching or other informative spoken information and the like. For this purpose, the smartphone is equipped with integrated loudspeakers 306. Alternatively or simultaneously, a headphone connection may be available to allow a headphone worn by the patient to be connected.

Preferably, the display screen 302 is a touchscreen and as such does not only serve to display information but also may be used as a user interface to obtain user input, particularly input from the patient.

Accordingly, the smartphone 300 allows a patient to listen to sound sequences of his/her scheduled medical procedure in advance. To this end, procedural information is provided. Using the touchscreen as a user interface the user may further provide sound manipulation information. For example, the user may change the volume of the sound sequence using a button displayed on the display screen 302. However, there may be provided other options for the patient to manipulate the sound sequences presented. For example, there could be the option that the patient changes the length of a sound sequence.

Additionally, the smartphone may display several entertainment program components, configured to accompany the sound sequences, the patient can choose to listen and/or watch. For example, when the sound sequences conveyed are perceived as unpleasant by the patient he/she may request (via the touchscreen) to have his/her favorite music or sounds of soothing rustling leaves be played during these sound sequences. To this end, several manipulation buttons may be displayed on the display screen. However, it is also conceivable that the smartphone 300 further comprises a microphone to obtain such information/user input.

The device 10 implemented as a smartphone 300 or other handheld device allows a patient to get familiar with the sounds of his/her medical procedure wherever he/she likes, particularly in places where they feel comfortable. Therefore, the device is preferably accessible at home for the patient. In fact, it is expected that patients need some time to play with the device and its options to adjust their sequence and accompanying entertainment program.

The implementation of the device 10 shown in Fig. 4 represents a simple implementation. However, more elaborate options, apart from changing volume and the presentation of audio/video content as shown are conceivable.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

## Claims

1. Device (10) for personalized noise preparation of a patient scheduled to undergo a medical procedure, the device comprising:
an input (12) configured to
obtain procedural information comprising information about the scheduled
medical procedure;
a processor (14) configured to
- obtain and/or generate sound sequence information comprising
information about sound sequences associated with sequences of the scheduled medical procedure based on the obtained procedural information;
- select and order the sound sequence information, particularly in accordance with the course of the medical procedure, based on the procedural information; and
- generate control signals for controlling a rendering unit (30) to render the sound sequences in accordance with the selected and ordered sound sequence information; and
an output (16) configured to output the control signals to the rendering unit (30).

2. Device (10) according to claim 1,
wherein the processor (14) is configured to select and order the sound sequence information, based on the procedural information, to generate the control signals for controlling the rendering unit (30) to render the sound sequences to match the acoustic pattern, pitch, pace, length and/or order of sound sequences generated by a medical device used in the course of the scheduled medical procedure.

3. Device (10) according to claim 2,
wherein the processor (14) is configured to obtain calibration information, comprising information for calibrating the processor (14) to adapt the sound sequence information in accordance with specific sound sequence features related to specifics of the medical device used in the course of the medical procedure, the specifics comprising any one of type, age, condition, use frequency, environment of the medical device, and/or in accordance with sample sound sequences from the medical device, and to adapt the sound sequence information based on the calibration information.

4. Device (10) according to any one of the preceding claims,
wherein the processor (14) is configured to obtain sound manipulation information, comprising information about a request of the patient for manipulation of the sound sequences, wherein the sound manipulation information comprises information on any one of changing volume, acoustic pattern, pitch, pace, length, selection and/or order of one or more of the sound sequences, and to adapt the sound sequence information in accordance with the sound manipulation information.

5. Device (10) according to any one of the preceding claims,
wherein the processor (14) is further configured to obtain and/or generate one or more entertainment program components, comprising one or more of an audio entertainment component, a video entertainment component and/or an interactive entertainment component, based on the procedural information, and to generate entertainment control signals for controlling the rendering unit (30) to render the one or more entertainment program components based on the entertainment program components;
wherein the output (16) is configured to output the entertainment control signals to the rendering unit (30).

6. Device (10) according to claim 5,
wherein the processor (14) is configured to obtain entertainment manipulation information comprising information about a request of the patient for manipulation of the entertainment program components, wherein the entertainment manipulation information comprises information on any one of changing type, theme, volume, acoustic pattern, pitch, pace, length, selection and/or order of the one or more audio entertainment components, changing any one of type, theme, display size, resolution, brightness, contrast, coloring, length, selection and/or order of the video entertainment components and/or changing any one of type, theme, level of interactivity, length, selection and/or order of the interactive entertainment components, and to adapt the one or more entertainment program components in accordance with the entertainment manipulation information.

7. Device (10) according to claim 5 or 6,
wherein the processor is configured to further obtain patient information comprising information regarding the patient, and wherein the processor (14) is configured to additionally use the patient information for obtaining, generating and/or adapting the one or more entertainment program components.

8. Device (10) according to claims 5 to 7,
wherein the obtained one or more entertainment program components comprise one or more labels and/or wherein the processor (14) is configured to assign one or more labels to the obtained and/or generated one or more entertainment program components, wherein the processor (14) is configured to assign the one or more labels by using a label database or a labeling program relating the one or more labels to the one or more entertainment program components, and wherein the processor (14) is configured to select and/or order the one or more entertainment programs based on their labels and to generate the entertainment control signals based on the selected and/or ordered one or more entertainment program components.

9. Device (10) according to any one of the preceding claims,
wherein the input (12) is configured to obtain, as procedural information, information regarding one or more of
- the type of medical procedure,
- medical device used in the medical procedure;
- the body part of the patient to undergo the medical procedure,
- duration and/or time of the medical procedure,
- course of the medical procedure.

10. Device (10) according to any preceding claim,
wherein the input (12) is configured to obtain the procedural information from one or more of a user interface (40) configured to obtain user input, an exam card, a medical record, a procedural information database.

11. System (100) for personalized noise preparation of a patient scheduled to undergo a medical procedure, the system (100) comprising:
- a device (10) for personalized noise preparation of a patient scheduled to undergo a medical procedure according to any one of the preceding claims; and
- a rendering unit (30) configured to render sound sequences according to the control signals obtained from the device (10).

12. System (100) according to claim 11, further comprising one or more of:
- a user interface (40) configured to obtain user input of the sound sequence information, procedural information, calibration information, patient information, sound manipulation information and/or entertainment manipulation information;
- a sound sequence information database (60) comprising sound sequence information comprising information about sound sequences associated with sequences of the scheduled medical procedure;
- a procedural information database (56) comprising procedural information comprising information about the scheduled medical procedure;
- a calibration information database (54) comprising calibration information, the calibration information comprising information for calibrating the processor of the device to adapt the sound sequence information in accordance with specific sound sequence features related to specifics of the medical device used in the course of the medical procedure;
- a patient information database (52) comprising patient information comprising information regarding the patient;
- an entertainment program components database (58) comprising a plurality of entertainment program components, in particular comprising one or more of an audio entertainment component, a video entertainment component and/or an interactive entertainment component.

13. System (100) according to claim 11 or 12,
wherein the system (100) is implemented in a handheld device, in particular a smartphone, laptop or tablet, and/or in a computer or a game console;
wherein the rendering unit (30) is implemented as a display configured to display visual information to the patient and/or as a loudspeaker configured to output audible information to the patient.

14. Method for personalized noise preparation of a patient scheduled to undergo a medical procedure, the method comprising:
obtaining procedural information comprising information about the scheduled medical procedure;
obtaining and/or generating sound sequence information comprising information about sound sequences associated with sequences of the scheduled medical procedure based on the obtained procedural information;
selecting and ordering the sound sequence information, particularly in accordance with the course of the medical procedure, based on the procedural information;
generating control signals for controlling a rendering unit (30) to render the sound sequences in accordance with the selected and ordered sound sequence information; and
outputting the control signals to the rendering unit (30).

15. Computer program comprising program code means for causing a computer to carry out the steps of the method as claimed in claim 14 when said computer program is carried out on the computer.
